# EUROPEAN PATENT APPLICATION

(11) **EP 0 675 195 A1**
(43) Date of publication of application: **04.10.1995**
(21) Application number: 95103219.2
(22) Date of filing: 07.03.1995
(51) Int. Cl.: C12M 1/18

(54) **Method to fill slides with culture media and slides with culture media obtained with this method**

(30) Priority: 28.03.1994 IT UD940046
(71) Applicant: TAPPITAL Srl, I-15100 Alessandria (IT)
(72) Inventor: Caruana, Alfonso, I-15068 Pozzolo Formigaro (AL) (IT)
(74) Representative: Petraz, Gilberto Luigi

(57) **Abstract**

Method to fill slides with culture media, the slides (10) being used for microbiological analysis of samples of liquids or surfaces and including a plurality of compartments (15) each of which is suitable to hold a specific culture medium, the compartments (15) being defined by at least three raised lateral edges (13-14), the slide (10) cooperating with closure and containing means (12-20) after consolidation of the media, each medium being poured from above by an appropriate pouring beaker (28), the slide (10) being kept in a vertical position within a flask (11) having the function of a mould.

Slide (10) with culture media, which is used for microbiological analysis of samples of liquids or surfaces, and includes a plurality of compartments (15) each of which is suitable to hold a specific culture medium, the compartments (15) being defined by at least three raised lateral edges (13-14), the slide (10) cooperating with closure and containing means (12-20) after consolidation of the media, the culture media being positioned in their specific compartments (15) according to one or another method of the claims.

## Description

This invention concerns a method to fill slides with culture media and concerns also the slides with culture media obtained with this method, as set forth in the respective main claims.

The invention is applied to the field of pre-arrangement of slides to hold the culture media, the slides being used for the conducting of microbiological analyses of samples of liquids, such as urine for instance, or of any type of surface for the purpose of ascertaining the presence and level of bacteriological contamination of such samples.

Slides holding culture media and used in the field of microbiological analyses are known in the state of the art.

They consist typically of a thin strip of plastic having at its sides raised edges to define seats to hold the culture media.

The plastic strip may possibly be divided on one or both of its faces by lengthwise, transverse or oblique intermediate raised partitions into two or more compartments to be filled with a plurality of different media. In this connection reference should be made, for instance, to WO-A-9307254.

Slides which contain altogether three or five compartments are typically most often used.

Each different culture medium is employed to enhance the growth of a particular microcellular organism, the presence of which the bacteriological analysis is intended to verify.

The end of the slide generally includes knurled portions and/or retaining teeth to retain the culture medium after the latter has been poured and while it is becoming consolidated.

The slides are normally equipped on their upper end with means for attachment, by pressure or with a spring catch for instance, to a threaded stopper, which is then clamped on a containing flask with which the slides, when filled with the culture media, are sent to their end user, as can be seen, for instance, in Fig.3 of FR-A-2.264.089 or in Fig.8 of EP-A-0111783.

The flask serves as a container to protect the slide during transport and handling of the same and also after performance of the analysis and during the period required for growth of the bacteria.

The process of filling the slides with the media in the state of the art is carried out by making the means which pour the culture medium or media in a semi-liquid or gelatinous state cooperate with the slide placed in a strictly horizontal position.

The slide is filled first on one side; then consolidation of the medium is awaited and thereafter the slide is overturned and is filled on its other side.

This process obviously entails several problems; firstly it involves a considerable time for its completion inasmuch as the filling of the two sides of the slide is always separated into two periods with an interval for consolidation of the first media poured.

Several passes may also be needed for the same face of the slide to have the filling of its compartments completed.

Moreover, it is necessary to ensure a strictly horizontal positioning of the slide so as to obviate fluctuations of the medium in a gelatinous state with a resulting build-up at one side of the compartment allotted to the medium or even possible overflowing into the next compartment, if there is one.

Furthermore, the outer containing flask does not protect the medium fully against the action of external agents, nor does it prevent contamination by pollutants nor possible formation of condensate within the flask itself.

This situation is very important with coloured media where a drop of condensate thus formed may contaminate the adjacent medium and may cause a mixture of colours that renders identification of the media difficult.

US-A-3,816,045 discloses a system for placing a layer of gelatinous material, such as a medium for biological analyses for instance, on a support in the form of a sheet by pouring from above.

This pouring system is achieved by making the sheet-shaped support cooperate with two opposed elements, one of which has the function of a mould while the other has the function of a countermould, within which the sheet-shaped support is positioned.

This system includes a stationary filling structure and can be employed only for supports in the form of a film or sheet, as can be seen in Fig.1 of US-A-3,816,045.

The present applicants have therefore tackled the problem of reducing the times and complexity of the filling process and of obtaining an end product safe against any contamination by external polluting agents, and for this purpose have investigated, tested and accomplished this invention.

This invention is set forth and characterised in the respective main claims, while the dependent claims describe variants of the idea of the main solution.

The method of filling the slides with the relative culture media according to the invention enables the process of pouring the media to be speeded up and simplified considerably.

The method also enables the superficial morphology of the culture media on the slides to be configured as desired.

Moreover, the method makes possible the obtaining of a product which can be directly assigned to an end user and which possesses excellent characteristics of safety against contamination by polluting agents.

According to the invention the filling of the slides with the relative culture media takes place with the slide kept in a vertical position.

For this purpose the slide according to the invention is equipped with compartments formed by raised end edges and raised lateral edges, which extend along the whole usable length of the slide.

According to a variant the compartments are formed by raised edges on three sides, the upper side being upwardly open so as to facilitate filling.

According to a preferred embodiment of the invention the slide has a substantially cylindrical conformation, the compartments being defined by lengthwise ribs and having a base consisting of a substantially curved surface.

According to a variant the slide has a substantially rectangular flattened conformation, with the compartments defined by the raised lateral edges and possible also by intermediate raised edges where there are a plurality of compartments defined on one surface of the slide.

In this case all the compartments have a substantially flat base.

According to the invention all the compartments of the slides are filled in one single operation by using suitable means including beakers suitably arranged and oriented to pour the media.

Moreover, special checking of the position of the slide during the filling is not required since the medium adapts itself to the conformation of the compartment without particular problems.

According to the invention the filling takes place by positioning the slide within a first flask which has a size substantially mating with the length of the compartments of the slide and which acts as a mould for the filling; the slide is left within the first flask during the necessary consolidation of the medium.

The inner conformation of this first moulding flask makes possible an assurance of a required efficient filling of the compartments and an excellent retention of the media within the compartments.

In a preferred embodiment of the invention the first flask is shaped with rounded portions inside so as to fit against the meniscus which the medium, having been poured in a gelatinous state, forms in the compartment.

In another embodiment the first flask is shaped inside with increased rounded portions so as to shape the upper part of the culture medium with a cylindrical development in order to facilitate bacteriological analysis of flat surfaces performed by pressure of the medium against the surfaces themselves.

According to the invention the slide together with the first flask is then inserted into a second containing flask.

According to the state of the art the second containing flask is associated with the slide by means of a screw stopper, to which possible attachment elements of the slide are clamped, for instance by pressure or a spring catch or in another desired manner.

The edges of the slide which define the compartments can divaricate, according to a variant, towards the inside of the compartments to ensure an effective retention of the medium when the latter has become consolidated.

Where the compartments have one upper side open upwards, this conformation is easy to embody by using a male mould which can be withdrawn from above.

Moreover, the edges can be embodied with a material which can be deformed at least partly and can be at least partly formed with a pointed edge so as to provide a tapered seal engagement against the sidewalls of the first flask during the filling step.

This embodiment ensures great safety against possible external contamination of the culture media by polluting agents, condensate or other means and ensures also great ease, speed and accuracy of the filling operations.

In another embodiment of the invention, which is suitable in the event of mechanisation of the whole filling process, the first filling flask acts only as a mould during the filling process; a plurality of these stationary moulds/flasks are included and are inserted, for instance, into a filler turntable mechanism.

The slides are introduced into these moulds, are filled with the relative media, are then withdrawn after consolidation of the media and are possibly inserted and clamped in the relative second containing flasks by means of suitable stoppers.

According to a variant of the invention a space may be provided between two adjacent compartments and be kept empty to prevent contamination between two media.

According to another variant of the embodiment, when the product is sent for use together with the first flask, the second containing flask may include lateral guides in which are inserted suitable fins on the sides of the first flask.

This embodiment ensures a better insertion and a better seal engagement of the slide in the second containing flask and prevents undesired movements of the slide during the period of consolidation of the medium or during the period of bacteriological culture.

In this variant the closure of the stopper against the second containing flask takes place not by screw closure but with a spring catch system, of a bayonet or another analogous type, for instance.

The attached figures are given as a non-restrictive example and show a preferred embodiment of the invention as follows:-
- Fig.1: shows a possible layout of the method according to the invention;
- Fig.2: shows a lengthwise section of a possible form of embodiment of the slide/containing-flasks assembly employed in the method of filling from above according to the invention;
- Fig.3: is a plan view of the assembly of Fig.1 without the stopper;
- Fig.4: shows the first flask according to the invention;
- Fig.5: shows the second containing flask according to the invention;
- Fig.6: shows a knock-down diagram of the assembly of Fig.1 without the second containing flask;
- Fig.7: shows a plan view of a cross-section of a possible slide for the culture media according to the invention.

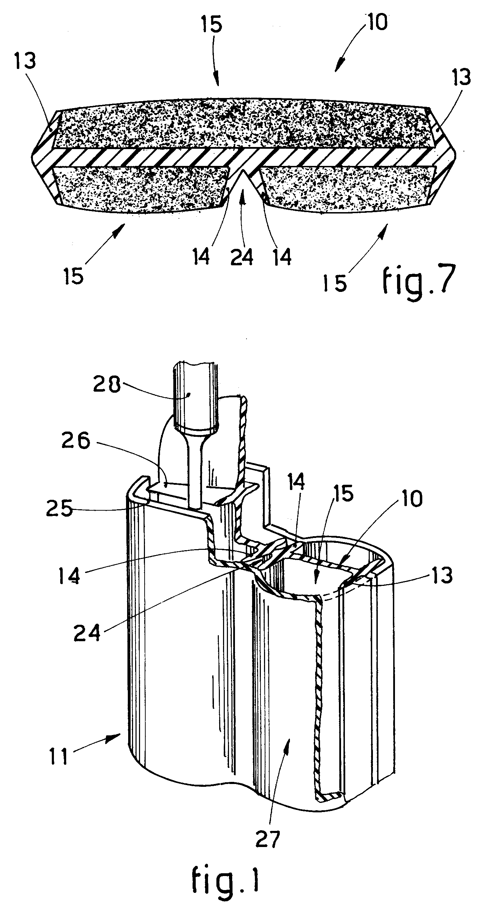

Fig.1 shows one of the possible forms of obtaining the method according to the invention.

The description which follows has been drawn up with regard to slides 10 having a substantially elliptic form, but it is to be understood that the invention is applied also to slides 10 having a circular, rectangular, square or other desired form.

The method provides for the filling of a slide 10 inserted into a first flask 11, which acts as a mould, by means of the pouring of culture media within relative compartments 15 defined on the slide 10.

The first flask 11 is made advantageously of a plastic material but can also be made of another material such as a metallic material, for instance.

In this case the pouring is carried out from above by using appropriate beakers 28, of which only one is shown in the figure for the sake of simplicity of description; the beakers 28 are introduced into the space between the upper edge 25 of the first flask 11 and the upper edge 26 of the slide 10 delimiting the height of the compartment 15.

According to the invention the same number of beakers 28 is used as the number of compartments 15 to be filled in carrying out the filling of the slide 10 at the same time in one single step.

The first flask 11 has advantageously rounded sidewalls 27 for formation of the meniscus of the culture medium poured in a gelatinous state. This enables a culture medium to be consolidated with a rounded conformation, which is especially efficient for use in the event of bacteriological analyses of surfaces when the medium has to be pressed against the surface to be analysed.

Raised lateral and end edges 13 and possible intermediate raised partitions 14 which define the compartments 15 in the lengthwise direction have advantageously a conformation divaricating towards the inside of the compartments 15 so as to enhance retention of the medium poured into the compartments 15.

The edges 13 and intermediate partitions 14 have also a pointed conformation to provide a seal engagement against the inner sidewalls of the first flask 11.

A separating space 24 is included advantageously between one compartment 15 and the next one on the same face of the slide 10 so as to avoid contamination between two different media.

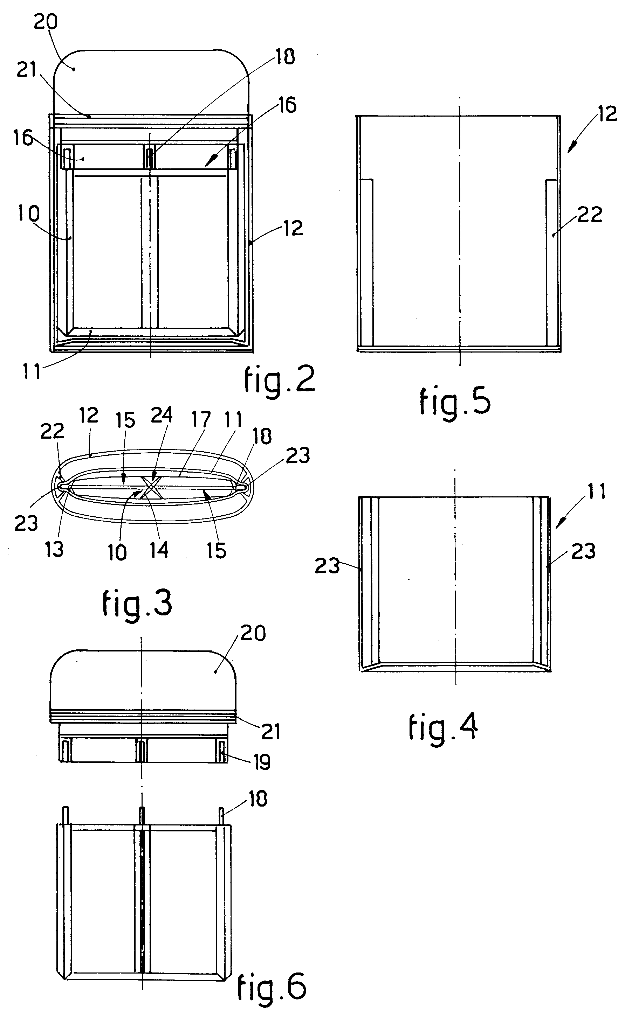

Fig.2 shows a possible form of embodiment of the slide 10 according to the invention when associated with a first flask 11, or mould for the filling, and when inserted together with the first flask 11 into a second containing flask 12.

In the case of the slide 10 of Fig.2 each compartment 15 has its upper end 16 open upwards to assist filling of all the compartments 15 from above at the same time in one single step by means of the pouring beakers 28.

In this case the slide 10 includes on its upper side attachment means 18 (Fig. 6) which cooperate, by being inserted with pressure, with mating hollows 19 in a closure stopper 20 of the slide 10.

The stopper 20 comprises ribs 21 which enable the stopper 20 to be clamped to the second containing flask 12, in which the slide 10 is sent to its end user. In this example the second containing flask 12 includes lateral guides 22 into which are inserted suitable fins 23 provided at the sides of the first flask 11 so as to ensure accurate insertion and clamping of the two flasks 11-12 to each other without rotation.

In this case the stopper 20, which is secured to the second containing flask 12 and to which the slide 10 is clamped by insertion of its own attachment means 18, includes spring-catch fixture means.

The first flask 11 comprises advantageously inner rounded portions 17 to enable the meniscus of the poured medium to be formed in the gelatinous state and the surface conformation of the medium to be determined in a required manner.

## Claims

1. Method to fill slides with culture media, the slides (10) being used for microbiological analysis of samples of liquids or surfaces and including a plurality of compartments (15) each of which is suitable to hold a specific culture medium, the compartments (15) being defined by at least three raised lateral edges (13-14), the slide (10) cooperating with closure and containing means (12-20) after consolidation of the media, the method being characterised in that each medium is poured from above by an appropriate pouring beaker (28), the slide (10) being kept in a vertical position within a flask (11) having the function of a mould.

2. Method as in Claim 1, in which the flask (11) having the function of a mould belongs, as a stationary part, to an automatic filling machine that fills slides (10).

3. Method as in Claim 1 or 2, in which the flask/mould (11) has sidewalls (27) which are at least partly rounded.

4. Method as in any claim hereinbefore, in which all the compartments (15) of a slide (10) are filled in one single operation.

5. Method as in any claim hereinbefore, in which the slide (10) is sent together with the first flask (11) to its end user.

6. Slide (10) with culture media, which is used for microbiological analysis of samples of liquids or surfaces, and includes a plurality of compartments (15) each of which is suitable to hold a specific culture medium, the compartments (15) being defined by at least three raised lateral edges (13-14), the slide (10) cooperating with closure and containing means (12-20) after consolidation of the media, the slide being characterised in that the culture media are positioned in their specific compartments (15) according to one or another method of the claims hereinbefore.

7. Slide (10) with culture media, which has all the compartments (15) belonging to the same face of the slide arranged lengthwise side by side;

8. Slide (10) as in Claim 7, in which all the compartments (15) have their upper end (16) open upwards.

9. Slide (10) as in Claim 7 or 8, in which the raised edges (13-14) have a pointed conformation and divaricate towards the inside of the compartment (15).

10. Slide (10) as in any of Claim 7 to 9 inclusive, in which a separating zone (24) to prevent contamination is included between two adjacent compartments (15).
